# EUROPÄISCHE PATENTSCHRIFT

(11) **EP 4 048 176 B1**
(45) Veröffentlichungstag und Bekanntmachung des Hinweises auf die Patenterteilung: **20.09.2023**
(21) Anmeldenummer: 20800566.0
(22) Anmeldetag: 23.10.2020
(51) Int. Cl.: A61B 18/12, A61B 18/00, A61B 17/00

(54) **ELEKTROCHIRURGIE-GENERATOR**
ELECTROSURGICAL GENERATOR
GÉNÉRATEUR ÉLECTROCHIRURGICAL

(30) Priorität: 24.10.2019 DE 102019128792
(43) Veröffentlichungstag der Anmeldung: 31.08.2022
(73) Patentinhaber: Olympus Winter & Ibe GmbH, 22045 Hamburg (DE)
(72) Erfinder: DIETRICH, Stefan, 12169 Berlin (DE); DIJKSTRA, Jelle, 10781 Berlin (DE); FAEHSING, Thomas, 12305 Berlin (DE)
(74) Vertreter: Eisenführ Speiser
(86) Internationale Anmeldenummer: PCT/EP2020/079951
(87) Internationale Veröffentlichungsnummer: WO 2021/078969

(56) Entgegenhaltungen:
- WO-A1-2004/045436
- DE-A1- 2 901 153
- DE-A1-102015 201 470
- US-A- 4 712 544
- US-A1- 2016 074 093

## Beschreibung

Die Erfindung betrifft einen Elektrochirurgie-Generator mit Ausgangsanschlüssen zum Anschließen wenigstens eines Elektrochirurgie-Instruments, wobei die Ausgangsanschlüsse mit einer Sekundärwicklung eines Ausgangstransformators verbunden sind.

Mittels Elektrochirurgie kann biologisches Gewebe - also Körpergewebe - geschnitten, koaguliert (verödet) und/oder vaporisiert werden. Für die Elektrochirurgie werden typischerweise hochfrequente Wechselströme mit einer Frequenz zwischen 0,2 MHz und 3 MHz verwendet.

Ein Elektrochirurgiesystem umfasst in der Regel einen Elektrochirurgie-Generator zum Erzeugen des hochfrequenten Wechselstroms. Der Elektrochirurgie-Generator hat in der Regel zwei Ausgangsanschlüsse, an die ein Elektrochirurgie-Instrument angeschlossen werden kann und zwischen denen im Betrieb eine hochfrequente Wechselspannung bereitgestellt wird. Die Ausgangsanschlüsse sind einem Ausgangstransformator auf dessen Sekundärseite elektrisch verbunden. Zum Erzeugen einer hochfrequenten Wechselspannung bzw. hochfrequenten Wechselstroms zum Betreiben eines an die Ausgangsanschlüsse angeschlossenen Elektrochirurgie-Instruments weist der Elektrochirurgie-Generator eine HF-Generatoreinheit auf, die galvanisch mit einer Primärwicklung des Ausgangstransformators verbunden ist, so dass sich ein Schwingkreis bildet, dessen Bestandteil die Primärseite des Ausgangstransformators ist. Durch den Ausgangstransformator sind die Ausgangsanschlüsse und ein im Betrieb an diese angeschlossenes Elektrochirurgie-Instrument galvanisch von der HF-Generatoreinheit getrennt.

Ein derartiger Elektrochirurgie-Generator dient dazu, ein an die Ausgangsanschlüsse angeschlossenes Elektrochirurgie-Instrument mit einem hochfrequenten Wechselstrom zu versorgen, sodass mit dem Elektrochirurgie-Instrument eine gewünschte elektrochirurgische Anwendung wie das Schneiden oder eine Ablation von Körpergewebe möglich ist. Die verschiedenen elektrochirurgischen Anwendungen erfordern typischerweise hochfrequente Wechselströme, die sich je nach Anwendung hinsichtlich des Strombedarfs oder der Spannung unterscheiden. Daher stellen bekannte Elektrochirurgie-Generatoren typischerweise verschiedene Betriebsmodi zur Verfügung. Beispielsweise gibt es Betriebsmodi, in denen der Elektrochirurgie-Generator eine sehr hohe Spitzenspannung zwischen 4KV und 5KV erfordern, wie dies für bestimmte Koagulationen nötig ist. Ein anderer Betriebsmodus erfordert sehr hohe Ströme, beispielsweise das Schneiden von Gewebe bei Verwendung einer Salzlösung.

Um derartige, verschiedene Betriebsmodi zur Verfügung zu stellen, weisen bekannte Elektrochirurgie-Generatoren einen Ausgangstransformator auf, bei dem primärseitig und/oder sekundärseitig Wicklungen umgeschaltet werden können. Andere bekannte Elektrochirurgie-Generatoren weisen mehrere unabhängige Transformatoren auf, beispielsweise einen zum Erzeugen einer hohen Ausgangsspannung und einen anderen zum Bereitstellen eines hohen Ausgangsstroms. Ein Beispiel eines Elektrochirurgie-Generators mit drei Ausgangstransformatoren für drei verschiedene Betriebsmodi ist in DE 29 01 153 beschrieben. Ferner beschreibt US 2016/074093 A1 einen Elektrochirurgie-Generator mit einem Multi-Instrument-Modus.

Der Erfindung liegt die Aufgabe zu Grunde, einen Elektrochirurgie-Generator zu schaffen, der verschiedene Betriebsmodi bereitstellt und sich mit beschränktem Materialaufwand realisieren lässt.

Erfindungsgemäß wird diese Aufgabe durch einen Elektrochirurgie-Generator gelöst, der wenigstens zwei separate HF-Generatoreinheiten und wenigstens zwei separate Ausgangstransformatoren sowie insgesamt wenigstens vier Ausgangsanschlüsse zum Anschließen von wenigstens zwei Elektrochirurgie-Instrumenten aufweist. Mit der Sekundärwicklung eines jeweiligen Ausgangstransformators sind jeweils zwei Ausgangsanschlüsse derart verbunden, dass einer der Ausgangsanschlüsse dem Anschließen einer Arbeitselektrode dient und der andere der beiden Ausgangsanschlüsse zum Anschließen einer Neutralelektrode. Jede der HF-Generatoreinheiten bildet mit dem zugehörigen Ausgangstransformator und den entsprechenden Ausgangsanschlüssen je ein HF-Generatormodul.

Der erfindungsgemäße Elektrochirurgie-Generator weist eine Steuereinheit auf, die mit den wenigstens zwei HF-Generatormodulen verbunden und ausgebildet ist, folgende Betriebsmodi bereitzustellen:
- einen Parallelmodus, in dem wenigstens zwei Ausgangstransformatoren auf ihrer Sekundärseite parallel geschaltet sind, so dass ein Elektrochirurgie-Instrument über die Ausgangsanschlüsse mit einen höheren Ausgangstrom betrieben werden kann, als von einem HF-Generatormodul alleine bereitgestellt werden kann,
- einen Serienmodus, in dem wenigstens zwei Ausgangstransformatoren auf ihrer Sekundärseite in Serie geschaltet sind, so dass ein Elektrochirurgie-Instrument über die Ausgangsanschlüsse mit einer höheren Ausgangsspannung betrieben werden kann, als von einem HF-Generatormodul alleine bereitgestellt werden kann,und
- einen Multi-Instrument-Modus, in dem die Ausgangstransformatoren unabhängig voneinander mit jeweils einem Paar von Ausgangsanschlüssen für je ein Elektrochirurgie-Instrument verbunden sind. In einem Spezialfall können die beiden Neutralelektroden mit einander verbunden sein.

Optional kann die Steuereinheit ausgebildet sein, einen Multi-Elektroden-Modus bereitzustellen, bei dem ein Ausgangsanschluss eines Generatormoduls mit einem Ausgangsanschluss eines anderen Generatormoduls elektrisch verbunden ist, so dass sich insgesamt drei Ausgangsanschlüsse ergeben, an die jeweils eine Elektrode eines Elektrochirurgie-Instruments angeschlossen ist.

Wenn ein derartiger Elektrochirurgie-Generator beispielsweise zwei HF-Generatormodule aufweist, erlaubt deren erfindungsgemäßer Einsatz somit eine simultane Aktivierung zweier Ausgänge mittlerer Anforderung (Strom, Spannung, Leistung, z.B. 120W) und alternativ eine Aktivierung eines Ausganges mit hohem Strom oder hoher Spannung und/oder hoher Leistung.

Ein derartiger Elektrochirurgie-Generator weist hierfür wenigstens zwei unabhängige HF-Generatormodule auf, ohne dass diese überdimensioniert sein müssen oder einen zusätzlichen Bauteileaufwand erfordern, wobei die HF-Generatormodule - jeweils gebildet von einer HF-Generatoreinheit und zugehörigen Ausgangstransformator - mittels der Steuereinheit je nach Bedarf parallel oder in Serie geschaltet werden können. Anstatt somit beispielsweise zwei HF-Generatormodule aufzubauen, die jeweils sowohl den geforderten Maximalstrom als auch die geforderte Maximalspannung liefern können, sind erfindungsgemäß wenigstens zwei unabhängige HF-Generatormodule vorgesehen, die einen gewünschten Maximalstrom oder eine gewünschte Maximalspannung durch entsprechendes Verschalten der Ausgänge liefern können. In einer Standardanwendung (Single Output) können zwei HF-Generatormodule entweder in Serie oder parallelgeschaltet werden, um die notwendige Spannung oder den notwendigen Strom liefern zu können. In einem Dual-Instrument-Modus können die HF-Generatormodule unabhängig voneinander betrieben werden, allerdings jeweils nur mit dem halben Maximalstrom beziehungsweise der halben Maximalspannung, was für die meisten Anwendungen, in denen zwei Elektrochirurgie-Instrumente parallel eingesetzt werden sollen, ausreicht. Dadurch, dass die HF-Generatormodule von einer gemeinsamen Steuereinheit gesteuert werden, können die hochfrequenten Wechselströme auf der Sekundärseite der Ausgangstransformatoren insbesondere auch in einem Multi-Instrument-Modus aufeinander abgestimmt werden, um so beispielsweise Querströme zu minimieren. Außerdem erlaubt ein derartiger Elektrochirurgie-Generator auch das Betreiben von Elektrochirurgie-Instrumenten mit drei Elektroden.

Vorzugsweise weist der Elektrochirurgie-Generator wenigstens drei Schalter auf, die mit den Ausgangsanschlüssen von wenigstens zwei HF-Generatormodulen derart verbunden sind, dass für den Parallelmodus zwei Ausgangsanschlüsse verschiedener HF-Generatormodule für eine Arbeitselektrode und zwei Ausgangsanschlüsse der verschiedenen HF-Generatormodule für eine Neutralelektrode jeweils elektrisch miteinander verbunden werden können, in dem zwei der drei Schalter geschlossen werden und so die beiden HF-Generatormodule an ihren Ausgängen parallelgeschaltet werden. Für den Serienmodus kann ein Ausgangsanschluss für eine Arbeitselektrode eines Generatormodules und ein Ausgangsanschluss für eine Neutralelektrode eines anderen Generatormodules elektrisch miteinander verbunden werden, indem der dritte Schalter geschlossen wird und so die beiden HF-Generatormodule an ihren Ausgängen in Serie geschaltet werden. Für einen Dual-Instrument-Modus sind alle drei Schalter geöffnet und die beiden HF-Generatormodule können unabhängig voneinander betrieben werden. Dadurch, dass die beiden HF-Generatormodule jedoch auch in diesem Modus vorzugswiese von der einen Steuereinheit gesteuert werden, kann der Betrieb der wenigstens zwei grundsätzlich voneinander unabhängigen HF-Generatoreinheiten durch die Steuereinheit so aufeinander abgestimmt werden, dass beispielsweise Querströme vermieden werden oder dass ein Instrument mit mehr als zwei Elektroden sinnvoll betrieben werden kann.

Die Ansteuerung der Schalter und das entsprechende Umschalten der Schalter erfolgt vorzugsweise durch die Steuereinheit, die zu diesem Zweck mit den wenigstens drei Schaltern wirkverbunden ist und die Schalter somit passend zu einem jeweils gewählten Betriebsmodus öffnen oder schließen kann.

Die Schalter können mechanische Schalter wie beispielsweise Relais sein, aber auch von Halbleitern beispielsweise von Feldeffekttransistoren oder ähnlichem gebildet sein.

Wie bereits weiter vorne angedeutet, ist die Steuereinheit zum Steuern der HF-Generatoreinheiten vorzugsweise mit jeder der wenigstens zwei HF-Generatoreinheiten wirkverbunden. Auf diese Weise kann die Steuereinheit auch das jeweilige Erzeugen eines hochfrequenten Wechselstroms am Ausgang eines jeweiligen Ausgangstransformators beeinflussen, um beispielsweise die erzeugten hochfrequenten Wechselströme in der Phase aufeinander abzustimmen und so Querströme zu vermeiden.

Vorzugsweise ist die Steuereinheit weiterhin ausgebildet, zwei Varianten des Multi-Instrument-Modus bereitzustellen, nämlich eine erste Variante, in der die Ausgangsanschlüsse zum Anschließen einer Neutralelektrode nicht elektrisch miteinander verbunden sind und eine zweite Variante, in der die Ausgangsanschlüsse zum Anschließen einer Neutralelektrode elektrisch miteinander verbunden sind.

In einer Ausführungsvariante des Elektrochirurgie-Generators sind die wenigstens zwei Ausgangsanschlüsse zum Anschließen einer Neutralelektrode miteinander elektrisch verbunden und können beispielsweise einen gemeinsamen Neutral-Anschluss bilden.

Vorzugsweise sind die Ausgangstransformatoren jeweils primärseitig mit einer der HF-Generatoreinheiten derart verbunden, dass die jeweilige HF-Generatoreinheit zusammen mit dem zugehörigen Ausgangstransformator einen jeweiligen Schwingkreis bildet, wobei eine Primärwicklung des jeweiligen Ausgangstransformators Teil des entsprechenden Schwingkreises ist.

In einer weiteren vorteilhaften Ausführungsvariante ist die Steuereinheit ausgebildet, einen "Multi-Elektroden-Modus" bereitzustellen, bei dem ein Ausgangsanschluss eines Generatormoduls mit einem Ausgangsanschluss eines anderen Generatormoduls elektrisch verbunden ist, so dass sich insgesamt drei Ausgangsanschlüsse ergeben, an die jeweils eine Elektrode eines Elektrochirurgie-Instruments angeschlossen ist. In dem "Multi-Elektroden-Modus" kann entweder ein einzelnes Elektrochirurgie-Instrument mit drei Elektroden oder es können drei einzelne Sonden betrieben werden, wie sie beispielsweise bei der Tumorablation eingesetzt werden um ein großes Volumen zu koagulieren. Vorzugsweise sind in dem "Multi-Elektroden-Modus" die beiden Ausgangsanschlüsse zum Anschließen einer Neutralelektrode miteinander elektrisch miteinander verbunden.

Vorzugsweise ist die Steuereinheit ausgebildet, die wenigstens zwei HF-Generatormodule in dem "Multi-Elektroden-Modus" mit unterschiedlicher Phasenlage zu betreiben.

Die HF-Generatormodule sind vorzugsweise so ausgelegt, dass die maximale Ausgangsspannung je Generator-Modul 2500 Vₚₑₐₖ beträgt - und damit etwa die Hälfte der Maximalausgangsspannung von 4200 Vₚₑₐₖ.

Die HF-Generatormodule sind weiter vorzugsweise so ausgelegt, dass der maximale Ausgangsstrom 4,5 Aᵣₘₛ beträgt.

Schließlich sind die HF-Generatormodule vorzugsweise so ausgelegt, dass deren maximaler Frequenzbereich 40 kHz bis 1 MHz umfasst und somit das Spektrum von Ultraschall bis HF.

Die Steuereinheit ist vorzugsweise dazu ausgebildet, die Phasenlage zwischen den von den beiden HF-Generatormodule abgegebenen Strömen bzw. Spannungen zu steuern.

Die Erfindung soll nun anhand eines Ausführungsbeispiels mit Bezug auf die Figuren näher erläutert werden.
- Figur 1:: einen erfindungsgemäßen Elektrochirurgie-Generator mit zwei Generatormodulen;
- Figur 2:: illustriert den Betrieb des Elektrochirurgie-Generators aus Figur 1 in einem "Multi-Elektroden-Modus"; und
- Figur 3:: einen erfindungsgemäßen Elektrochirurgie-Generator mit drei Generatormodulen;
- Figur 4:: den Elektrochirurgie-Generator aus Figur 3 mit in Serie geschalteten Sekundärwicklungen aller drei Generatormodule;
- Figur 5:: den Elektrochirurgie-Generator aus Figur 3 mit parallel geschalteten Sekundärwicklungen aller drei Generatormodule;
- Figur 6:: den Elektrochirurgie-Generator aus Figur 3 mit in Serie geschalteten Sekundärwicklungen von zwei der drei Generatormodule;
- Figur 7:: den Elektrochirurgie-Generator aus Figur 3 mit parallelgeschalteten Sekundärwicklungen von zwei der drei Generatormodule; und
- Figur 8:: den Elektrochirurgie-Generator aus Figur 3 mit parallelgeschalteten Sekundärwicklungen von zwei der drei Generatormodule und einem dazu in Serie geschalteten dritten Generatormodul.

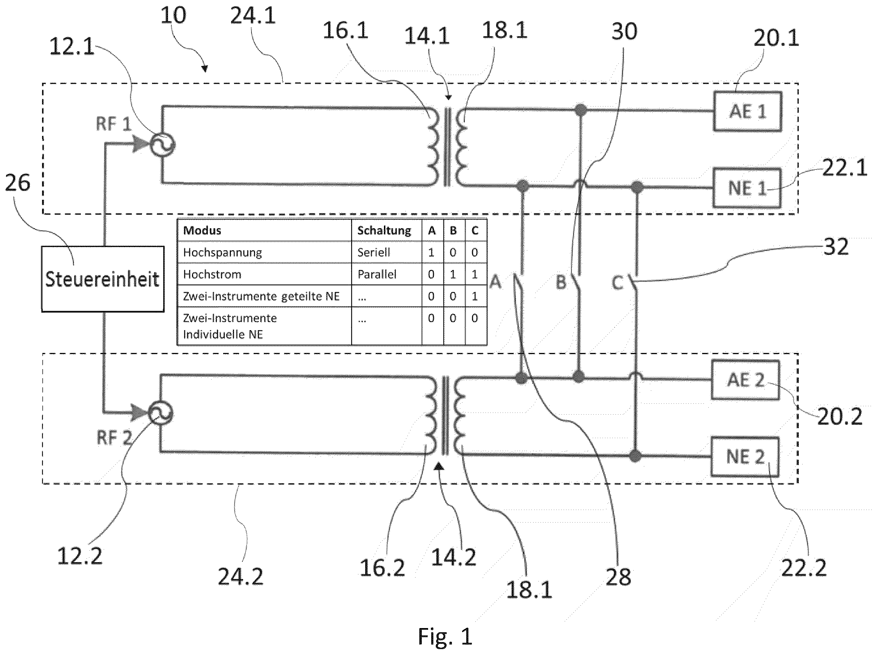

Figur 1 zeigt einen Elektrochirurgie-Generator 10, der zwei HF-Generatoreinheiten 12.1 und 12.2 und zwei Ausgangstransformatoren 14.1 und 14.2 aufweist.

Jeder der beiden Ausgangstransformatoren 14.1 und 14.2 weist auf der Primärseite jeweils eine Primärwicklung 16.1 beziehungsweise 16.2 und auf der Sekundärseite eine Sekundärwicklung 18.1 beziehungsweise 18.2 auf. Die jeweilige HF-Generatoreinheit 12.1 oder 12.2 ist mit der entsprechenden Primärwicklung 16.1 beziehungsweise 16.2 des zugehörigen Ausgangstransformators 14.1 beziehungsweise 14.2 verbunden, so dass jeweils eine HF-Generatoreinheit 12.1 oder 12.2 mit der zugehörigen Primärwicklung 16.1 beziehungsweise 16.2 des zugehörigen Ausgangstransformators 14.1 beziehungsweise 14.2 einen Schwingkreis bildet.

Mit der jeweiligen Sekundärwicklung 18.1 oder 18.2 des Ausgangstransformators 14.1 beziehungsweise 14.2 sind jeweils zwei Ausgangsanschlüsse 20.1 und 22.1 beziehungsweise 20.2 und 22.2 elektrisch verbunden. Von diesen Ausgangsanschlüssen bildet jeweils einer der Ausgangsanschlüsse einen Ausgangsanschluss 20.1 beziehungsweise 20.2 zum Anschließen einer Arbeitselektrode eines Elektrochirurgie-Instrumentes, während der jeweils andere Ausgangsanschluss 22.1 beziehungsweise 22.2 einen Ausgangsanschluss zum Anschließen einer Neutralelektrode eines jeweiligen Elektrochirurgie-Instrumentes bildet.

Eine jeweilige HF-Generatoreinheit 12.1 oder 12.2 bildet mit dem jeweils daran angeschlossenen Ausgangstransformator 14.1 beziehungsweise 14.2 und den zugehörigen Ausgangsanschlüssen 20.1 und 22.1 beziehungsweise 20.2 und 22.2 ein Generatormodul 24.1 beziehungsweise 24.2.

Beide HF-Generatormodule 24.1 und 24.2 sind grundsätzlich unabhängig voneinander, werden jedoch von einer gemeinsamen Steuereinheit 26 gesteuert. Die Steuereinheit 26 ist dazu sowohl mit der HF-Generatoreinheit 12.1 als auch mit der Generatoreinheit 12.2 wirkverbunden und kann so das jeweilige HF-Generatormodul 24.1 und 24.2 steuern.

Über insgesamt drei Schalter 28, 30 und 32 können die Ausgangsanschlüsse 20.1, 22.1, 20.2 und 22.2 der HF-Generatormodule 24.1 und 24.2 wahlweise zueinander parallelgeschaltet werden oder in Serie geschaltet werden. Dazu ist der Schalter 28 zum einen mit dem Ausgangsanschluss 22.1 des ersten Generatormoduls 24.1 zum Anschließen einer Neutralelektrode elektrisch verbunden, und zum anderen mit dem Ausgangsanschluss 20.2 zum Anschließen einer Arbeitselektrode des zweiten HF-Generatormoduls 24.2 elektrisch verbunden. Durch Schließen des Schalters 28 können die Ausgänge der beiden Generatormodule 24.1 und 24.2 in Serie geschaltet werden, sodass ein Instrument beispielweise an den Ausgangsanschluss 20.1 des ersten Generatormoduls 24.1 und den Ausgangsanschlüssen 22.2 des HF-Generatormoduls 24.2 angeschlossen werden kann und somit eine Spannung betrieben werden kann, die doppelt so hoch ist, wie die maximale Ausgangsspannung einer der HF-Generatormodule 24.1 oder 24.2. Zum Öffnen und Schließen des Schalters 28 ist dieser mit der Steuereinheit 26 wirkverbunden.

Schließen des Schalters 28 allein erlaubt es mit einem derartigen Elektrochirurgie-Generator auch das Betreiben von Elektrochirurgie-Instrumenten mit drei Elektroden. So kann zusätzlich zu den Betriebsmodi "Parallelmodus", "Seriellmodus" und "Multi-Instrument-Modus" auch ein "Multi-Elektroden-Modus" verwirklicht werden. Dieser kann sowohl mit einem einzelnen Instrument mit drei Elektroden genutzt werden oder auch mit drei einzelnen Sonden wie sie beispielsweise bei der Tumorablation eingesetzt werden um ein großes Volumen zu koagulieren.

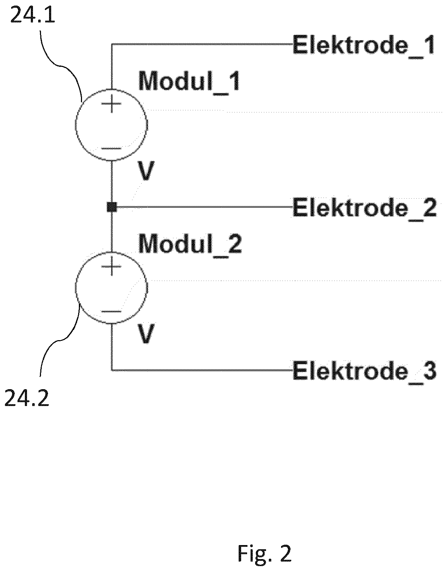

Wenn die Elektroden durch Schließen des Schalters 28 wie in Figur 2 dargestellt verschaltet werden, kann zwischen jedem Elektrodenpaar jede beliebige Spannung eingestellt werden. Wenn beispielsweise die Amplituden und Phasen der beiden HF-Generatormodule 24.1 und 24.2 gleich sind, liegt zwischen den Elektroden 1 und 2 bzw. 2 und 3 jeweils die einfache Spannung und zwischen den Elektroden 1 und 3 die doppelte Spannung an. Wenn die Amplituden gleich sind aber die Phase um 180° gedreht ist, liegt zwischen den Elektroden 1 und 2 bzw. 2 und 3 jeweils die einfache Spannung an und zwischen den Elektroden 1 und 3 keine Spannung.

Mithilfe der beiden Schalter 30 und 32 können die beiden HF-Generatormodule 24.1 und 24.2 zueinander parallelgeschaltet werden, damit ein Elektrochirurgie-Instrument mit einem Strom betrieben werden kann, der doppelt so groß ist, wie der Strom, den eines der beiden HF-Generatormodule 24.1 oder 24.2 alleine liefern kann. Zu diesem Zweck ist der Schalter 30 elektrisch mit den beiden Ausgangsanschlüssen 20.1 und 20.2 zum Anschließen der Arbeitselektrode eines Elektrochirurgie-Instrumentes verbunden, während der Schalter 32 mit den beiden Ausgangsanschlüssen 22.1 und 22.2 zum Anschließen an der jeweiligen Neutralelektrode eines Elektrochirurgie-Instrumentes elektrisch verbunden ist. Wenn beide Schalter 30 und 32 geschlossen und der Schalter 28 geöffnet ist, sind die beiden HF-Generatormodule 24.1 und 24.2 über ihre Ausgänge parallel geschaltet. Um dies bewirken zu können, sind auch die beiden Schalter 30 und 32 mit der Steuereinheit 26 wirkverbunden.

Wenn alle drei Schalter 28, 30 und 32 geöffnet sind, können die beiden HF-Generatormodule 24.1 und 24.2 unabhängig voneinander betrieben werden. Da jedoch die jeweiligen HF-Generatoreinheiten 12.1 und 12.2 jeweils mit der Steuereinheit 26 verbunden sind, kann die Steuereinheit 26 die beiden HF-Generatormodule 24.1 und 24.2 so auch in dem Modul-Instrument-Modus, in dem alle drei Schalter 28, 30 und 32 geöffnet sind, so steuern, dass die Spannungen über die Ausgangsanschlüsse 20.1 und 22.1 beziehungsweise 20.2 und 22.2 beispielsweise hinsichtlich der Faser so aufeinander abgestimmt sind, dass es bei einem Betrieb mit zwei Instrumenten nicht zu Querströmen kommt. Schließlich kann die Steuereinheit 26 durch Schließen alleine des Schalters 32 bewirken, dass die Neutralelektroden von zwei an den Elektrochirurgie-Generator 10 angeschlossenen Elektrochirurgie-Instrumenten das gleiche Potenzial haben.

Durch entsprechendes Schalten der Schalter 28, 30 und 32 kann die Steuereinheit 26 den Elektrochirurgie-Generator 10 also in insgesamt vier verschiedene Betriebsmodi schalten. Ein erster Betriebsmodus ist ein Serienmodus, in dem die beiden HF-Generatormodule 24.1 und 24.2 in Serie zueinander geschaltet sind. Dies ist dann der Fall, wenn die Steuereinheit 26 den Schalter 28 schließt und die beiden Schalter 30 und 32 öffnet. Ein weiterer Betriebsmodus ist ein Parallelmodus, in dem die beiden HF-Generatormodule 24.1 und 24.2 zueinander parallelgeschaltet ist. Um diesen Parallelmodus herzustellen, wird der Schalter 28 durch die Steuereinheit 26 geöffnet, während die beiden Schalter 30 und 32 durch die Steuereinheit 26 geschlossen werden. Wenn die Steuereinheit 26 alle drei Schalter 28, 30 und 32 öffnet, ergibt sich ein Dual-Instrument-Modus, bei dem die beiden HF-Generatormodule 24.1 und 24.2 auf ihrer Ausgangsseite elektrisch unabhängig voneinander sind. Wenn die Steuereinheit 26 alleine den Schalter 32 schließt, ergibt sich ein Dual-Instrument-Modus, bei dem die Ausgangsanschlüsse 22.1 und 22.2 zum Anschließen einer jeweiligen Neutralelektrode eines Elektrochirurgie-Instrumentes auf gleichem Potenzial liegen.

Die beiden HF-Generatormodule 24.1 und 24.2 sind so ausgelegt, dass die maximale Ausgangsspannung je Generator-Modul 2500 Vₚₑₐₖ beträgt - und damit etwa die Hälfte der Maximalausgangsspannung von 4200 Vₚₑₐₖ. Außerdem sind die beiden HF-Generatormodule 24.1 und 24.2 so ausgelegt, dass der maximale Ausgangsstrom 4,5 Aᵣₘₛ beträgt und dass deren Frequenzbereich 40 kHz bis 1 MHz umfasst und somit das Spektrum von Ultraschall bis HF.

Die Steuereinheit 26 ist dazu ausgebildet, die Amplitude und Phasenlage zwischen den von den beiden HF-Generatormodulen 24.1 und 24.2 abgegebenen Strömen bzw. Spannungen zu steuern.

Die Phasenlage wird somit durch die Steuereinheit 26 bestimmt und ist variabel. Wird an beide Generator-Module 24.1 und 24.2 beispielsweise ein Drei-Elektroden-Instrument (NE1/AE/NE2 - 3 elektrische Kontakte) angeschlossen, so kann durch eine Änderung der Phasenlage zwischen Schneide-Modus zum Gewebeschneiden (Cut) und einem Koagulations-Modus zum Koagulieren von Gewebe (Coag) gewechselt werden. Für den Koagulations-Modus beträgt der Phasenwinkel (die Phasenlage) 0° und für den Schneide-Modus 180°. Auch können zukünftige Instrumente von einer Phasenlagenänderung profitieren.

Der Elektrochirurgie-Generator 10 bietet somit eine Vielzahl von Betriebsmodi, und kann nach Bedarf entweder eine hohe Ausgangsspannung oder einen hohen Ausgangsstrom bereitstellen, ohne dass eine seiner HF-Generatormodule 24.1 oder 24.2 für die maximale, von dem Elektrochirurgie-Generator 10 bereitzustellende Ausgangsspannung oder den bereitzustellenden Ausgangstrom ausgelegt sein müssen.

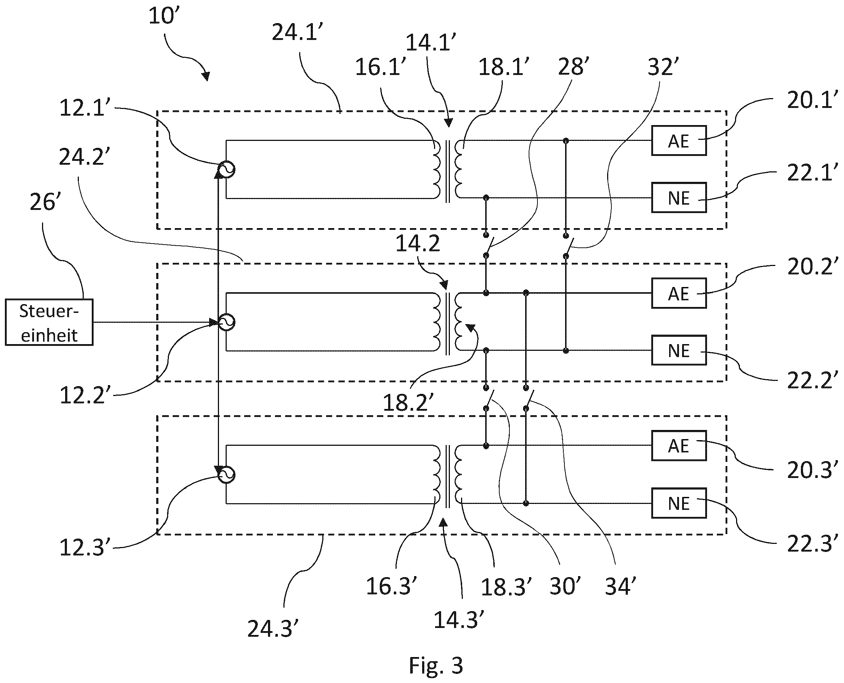

Ein Elektrochirurgie-Generator 10' kann auch mehr als zwei Generatormodule aufweisen, beispielsweise drei Generatormodule 24.1, 24.2' und 24.3'; siehe Figur 3.

Auch bei dem Elektrochirurgie-Generator 10' können die Ausgangstransformatoren 14.1', 14.2' und 14.3' sekundärseitig wahlweise in Serie geschaltet werden, parallelgeschaltet werden oder auch separat betrieben werden. Hierzu sind im Ausführungsbeispiel insgesamt vier Schalter vorgesehen, nämlich die Schalter 28`, 30`, 32' und 34`.

In Figur 3 sind sämtliche Schalter 28`, 30`, 32' und 34' geöffnet, sodass die drei Generator-module 24.1', 24.2` und 24.3' auf ihren Ausgangsseiten unabhängig voneinander sind. Die jeweils zugehörigen HF-Generatoreinheiten 12.1', 12.2' und 12.3' werden jedoch von der Steuereinheit 26' gesteuert, sodass ähnlich wie in dem Ausführungsbeispiel gemäß Figur 1 ein Phasenwinkel zwischen den durch die HF-Generatoreinheiten 12.1, 12.2 und 12.3 erzeugten hochfrequenten Wechselspannungen eingestellt werden kann.

Die Steuereinheit 26 ist im Übrigen auch mit dem Schalter 28`, 30`, 32' und 34' wirkverbunden, sodass diese Schalter durch die Steuereinheit 26 gesteuert, geschlossen oder geöffnet werden können.

In dem in Figur 3 dargestellten Beispiel sind - wie schon gesagt - sämtliche der vier Schalter 28`, 30`, 32' und 34' geöffnet.

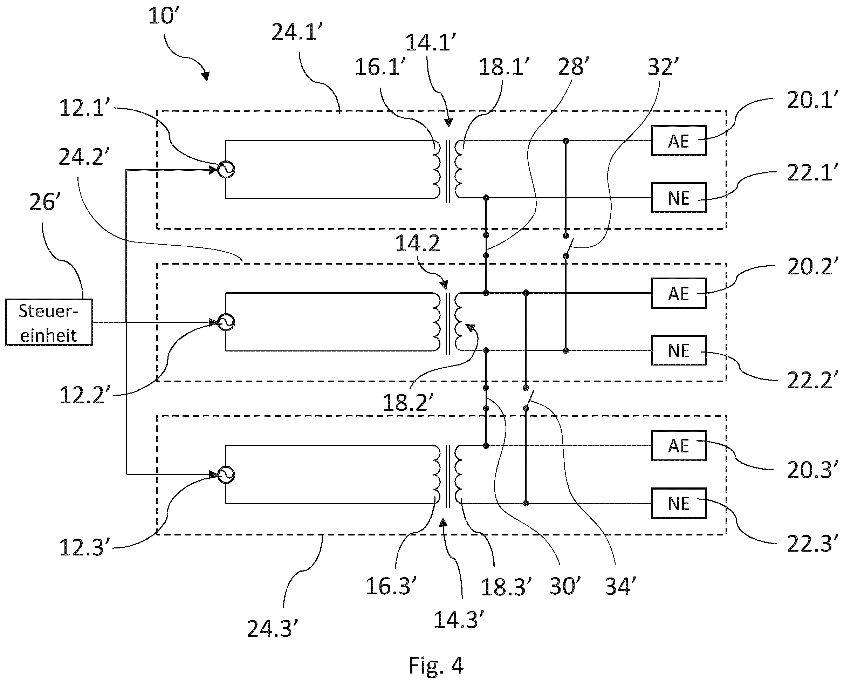

Wenn die Schalter 28` und 30' geschlossen, aber die Schalter 32' und 34' geöffnet sind, sind die drei Ausgangstransformatoren 14.1', 14.2' und 14.3' der Generatormodule 24.1', 24.2' und 24.3' auf ihrer Sekundärseite miteinander in Serie geschaltet. Dies bedeutet, dass die Sekundärwicklungen 18.1', 18.2` und 18.3` miteinander in Serie geschaltet sind, wenn die Schalter 28` und 30' geschlossen, aber die Schalter 32' und 34' geöffnet sind. Dies ist beispielhaft in Figur 4 abgebildet. Durch In-Serie-Schalten der Sekundärwicklungen 18.1', 18.2' und 18.3` liegt zwischen den Ausgangsanschlüssen 20.1 und 22.3 eine erhöhte Ausgangsspannung an, die beispielsweise in etwa der Summe der Ausgangsspannungen der einzelnen Ausgangstransformatoren 14.1', 14.2' und 14.3' entspricht. Um diese erhöhte Ausgangsspannung zu nutzen, kann ein Elektrochirurgie-Instrument an die Ausgangsanschlüsse 20.1' und 22.3' angeschlossen werden.

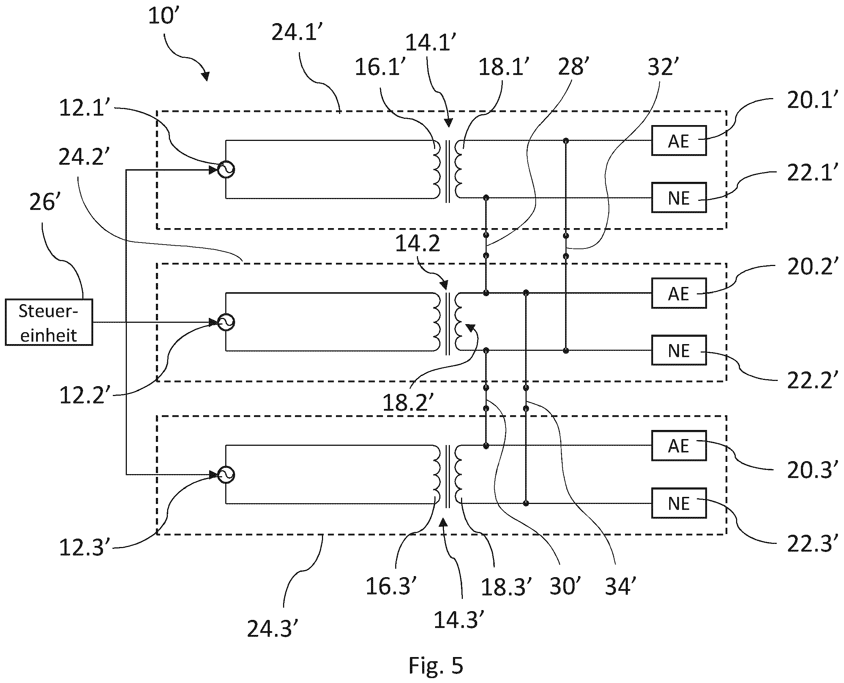

Um einen erhöhten Ausgangsstrom zu erzielen, können die drei Generatormodule 24.1', 24.2' und 24.3' ausgangsseitig auch parallelgeschaltet werden. Eine derartige Parallelschaltung liegt dann vor, wenn die Schalter 28`, 30`, 32' und 34' durch die Steuereinheit 26' gesteuert, sämtlich geschlossen sind. Dies ist beispielhaft in Figur 5 dargestellt. Wenn die Schalter 28`, 30', 32' und 34' geschlossen sind, sind die Sekundärwicklungen 18.1', 18.2` und 18.3' der Ausgangstransformatoren 14.1', 14.2' und 14.3` einander parallelgeschaltet. In dem dargestellten Ausführungsbeispiel wird die HF-Generatoreinheit 12.2' mit einer um 180° verschobenen Phasenlage betrieben, damit sich der gewünschte Effekt eines erhöhten Ausgangsstroms ergibt, denn durch die Anordnung der Schalter 28`, 30', 32' und 34' wird die Phasenlage des Ausgangstransformators 14.2' bei geschlossenen Schaltern in Bezug auf die die Phasenlagen der Ausgangstransformatoren 14.1' und 14.3' ebenfalls um 180° gedreht - , man kann sich bei dieser Verschaltung die Ausgangsanschlüsse 20.1 und 20.3 auf den Ausgangsanschluss 22.2 geklappt vorstellen, genauso wie die Ausgangsanschlüsse 22.1 und 22.3 auf den Ausgangsanschluss 20.2. Die Phasendrehung durch die geschlossenen Schalter 28`, 30', 32' und 34' kann durch entsprechendes Einstellen der Phasenlage an der HF-Generatoreinheit 12.2' kompensiert werden. Die HF-Generatoreinheit 12.2' wird daher bei geschlossenen Schaltern 28`, 30`, 32' und 34` gegenphasig zu den Generatoreinheiten 12.1' und 12.3' betrieben.

Um den erhöhten Ausgangsstrom zu nutzen, kann ein Elektrochirurgie-Instrument einerseits wahlweise an einen der Ausgangsanschlüsse 20.1', 22.2' oder 20.3' angeschlossen werden und anderseits an einen der Ausgangsanschlüsse 22.1', 20.2' oder 22.3'.

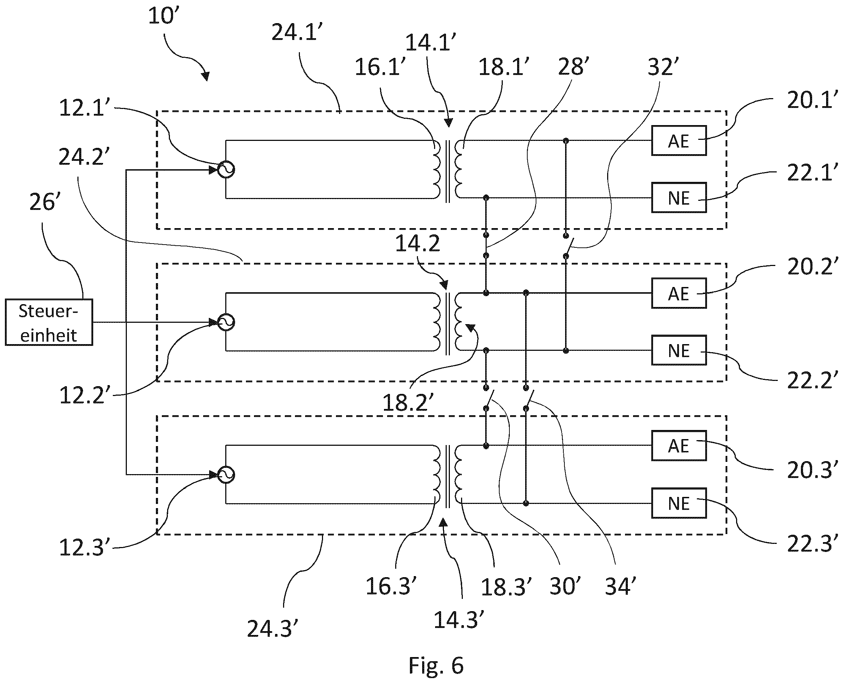

Wenn - wie in Figur 6 dargestellt - nur der Schalter 28` geschlossen ist, und die übrigen Schalter 30`, 32` und 34' geöffnet sind, sind nur die Generatormodule 24.1' und 24.2' ausgangsseitig in Serie geschaltet, während das dritte Generatormodul 24.3' unabhängig bleibt. In diesem Fall kann zwischen den Anschlüssen 20.1' und 22.2' ein Elektrochirurgie-Instrument angeschlossen werden, das eine erhöhte Ausgangsspannung benötigt, während ein anderes Elektrochirurgie-Instrument an die Ausgangsanschlüsse 20.3' und 22.3' angeschlossen werden können, um unabhängig von dem an die Ausgangsanschlüsse 20.1' und 22.2' angeschlossenen Elektrochirurgie-Instrument betrieben zu werden.

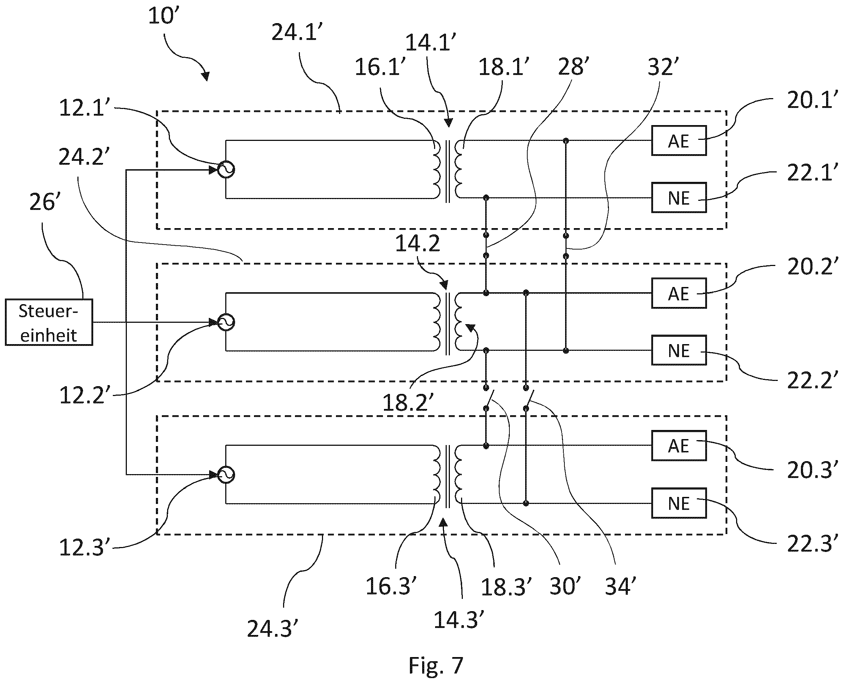

Um ein Elektrochirurgie-Instrument mit einem erhöhten Strombedarf in Kombination mit einem zweiten Elektrochirurgie-Instrument betreiben zu können, können beispielsweise auch die Generatormodule 24.1' und 24.2' ausgangsseitig parallelgeschaltet werden, während das dritte Generatormodul 24.3' unabhängig bleibt; siehe Figur 7. Um dies zu erreichen, werden die Schalter 28` und 32' durch die Steuereinheit 26' gesteuert geschlossen, während die Schalter 30' und 34' geöffnet bleiben. Auf diese Weise sind die Sekundärwicklungen 18.1' und 18.2' der Ausgangstransformatoren 14.1' oder 14.2` zueinander parallelgeschaltet. Auch hier müssen die HF-Generatoreinheiten 12.1' und 12.2' mit einer um 180° zueinander verschoben Phase - also gegenphasig - angesteuert werden, damit die Ausgangstransformatoren 14.1' und 14.2' auf Ausgangseite durch die Schalter 28' und 32' gleichphasig parallelgeschaltet werden. Beispielsweise wird die HF-Generatoreinheit 12.2' mit einer um 180° verschobenen Phasenlage betrieben.

Ein Elektrochirurgie-Instrument mit erhöhtem Strombedarf kann wahlweise einerseits an den Ausgangsanschluss 20.1' oder 22.2' angeschlossen werden und andererseits an den Ausgangsanschluss 20.2' oder 22.1'. Das zweite Elektrochirurgie-Instrument kann dann an die Ausgangsanschlüsse 20.3' und 22.3' angeschlossen werden.

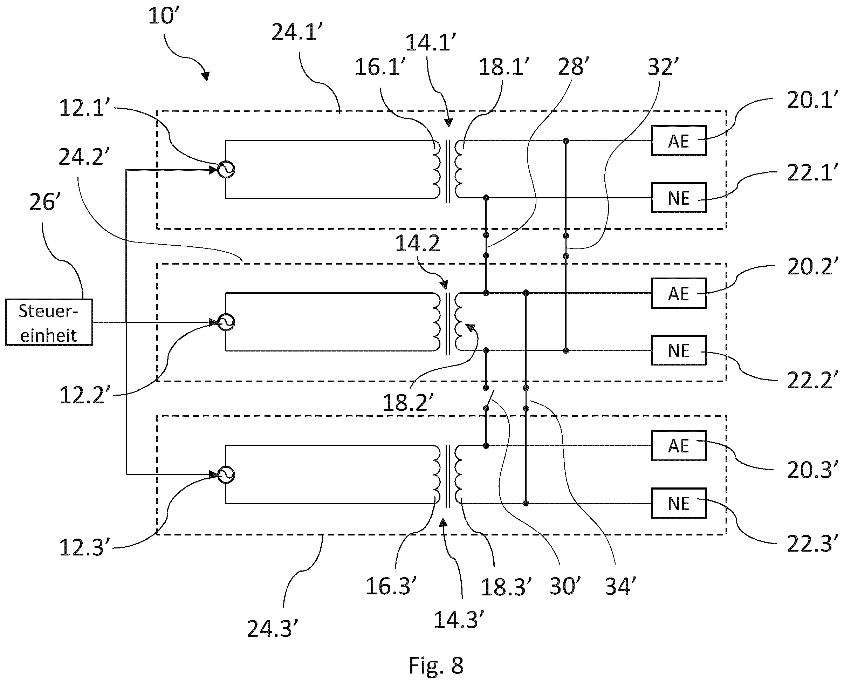

Auch ist es möglich, zwei der drei Generatormodule 24.1', 24.2' oder 24.3' parallel zu betreiben - beispielsweise die Generatormodule 24.1' und 24.2', wie dies in Figur 7 dargestellt ist - und das jeweils dritte Generatormodul - im Beispiel das Generatormodul 24.3' - dazu in Serie zu betreiben. Ein entsprechendes Beispiel in Figur 8 dargestellt.

Grundsätzlich ist es möglich, beliebige zwei der drei Generatormodule 24.1', 24.2' oder 24.3' parallel oder in Serie zu betreiben und das jeweils dritte Generatormodul unabhängig zu betreiben - analog zu den in den Figuren 6 und 7 dargestellten Ausführungsbeispielen. Dies kann insbesondere dann weitere Varianten ermöglichen, wenn die drei Generatormodule 24.1', 24.2' oder 24.3' nicht identisch sind, sondern z.B. unterschiedliche Ausgangsspannungen liefern.

### Bezugszeichenliste

- 10: Elektrochirurgie-Generator
- 12.1, 12.2, 12.3: HF-Generatoreinheiten
- 14.1, 14.2, 14.3: Ausgangstransformator
- 16.1, 16.2, 16.3: Primärwicklung
- 18.1, 18.2, 18.3: Sekundärwicklung
- 20.1, 22.1, 20.2, 22.2, 20.3, 22.3: Ausgangsanschlüsse
- 24.1, 24.2, 24.3: HF-Generatormodul
- 26: Steuereinheit
- 28, 30, 32, 34: Schalter

## Patentansprüche

1. Elektrochirurgie-Generator (10), umfassend wenigstens zwei HF-Generatoreinheiten (12.1; 12.2) und wenigstens zwei Ausgangstransformatoren (14; 14.2) mit je einer Primärwicklung (16.1; 16.2) und einer Sekundärwicklung (18.1; 18.2), von denen jeweils eine der Primärwicklungen (16.1; 16.2) mit einer der HF-Generatoreinheiten (12.1; 12.2) verbunden und eine der Sekundärwicklungen (18.1 ; 18.2) (14.1; 14.2) mit je zwei Ausgangsanschlüssen (20.1, 22.1; 20.2, 22.2) zum Anschließen eines Elektrochirurgie-Instruments verbunden ist, von denen jeweils einer der beiden Ausgangsanschlüsse (20.1; 20.2) zum Anschließen einer Arbeitselektrode und der jeweils andere der beiden Ausgangsanschlüsse (22.1; 22.2) zum Anschließen einer Neutralelektrode vorgesehen ist, wobei je eine HF-Generatoreinheit (12.1; 12.2) mit dem zugehörigen Ausgangstransformator (14.1; 14.2) und den entsprechenden Ausgangsanschlüssen (20.1, 22.1; 20.2, 22.2) ein HF-Generatormodul (24.1; 24.2) bildet, wobei die HF-Generatormodule (24.1; 24.2) mit einer Steuereinheit (26) verbunden sind, die ausgebildet ist, wenigstens folgende Betriebsmodi bereitzustellen:
- einen Parallelmodus, in dem die wenigstens zwei Ausgangstransformatoren (14.1, 14.2) parallel geschaltet sind, so dass ein Elektrochirurgie-Instrument über die Ausgangsanschlüsse (20.1, 22.1; 20.2, 22.2) mit einen höheren Ausgangstrom betrieben werden kann, als von einem HF-Generatormodul (24.1; 24.2) alleine bereitgestellt werden kann,
- einen Serienmodus, in dem die wenigstens zwei Ausgangstransformatoren (14.1, 14.2) in Serie geschaltet sind, so dass ein Elektrochirurgie-Instrument über die Ausgangsanschlüsse (20.1; 22.2) mit einer höheren Ausgangsspannung betrieben werden kann, als von einem HF-Generatormodul (24.1; 24.2) alleine bereitgestellt werden kann, und
- einen Multi-Instrument-Modus, in dem Ausgangstransformatoren (14.1, 14.2) unabhängig voneinander mit Anschlüssen für je ein Elektrochirurgie-Instrument verbunden sind.

2. Elektrochirurgie-Generator (10) nach Anspruch 1, **dadurch gekennzeichnet, dass** der Elektrochirurgie-Generator (10) mit dessen Steuereinheit (26) ausgebildet ist, zusätzlich einen Multi-Elektroden-Modus bereitzustellen, bei dem ein Ausgangsanschluss eines bereitzustellen s mit einem Ausgangsanschluss eines anderen Generatormoduls elektrisch verbunden ist, so dass sich insgesamt drei Ausgangsanschlüsse ergeben, an die jeweils eine Elektrode eines Elektrochirurgie-Instruments angeschlossen ist.

3. Elektrochirurgie-Generator (10) nach Anspruch 1 oder 2, **gekennzeichnet durch** wenigstens drei Schalter (28, 30, 32), die mit den Ausgangsanschlüssen (20.1, 22.1, 20.2, 22.2) derart verbunden sind, dass für den Parallelmodus zwei Ausgangsanschlüsse (20.1, 22.1, 20.2, 22.2) verschiedener Generatormodule für eine Arbeitselektrode und zwei Ausgangsanschlüsse (20.1, 22.1, 20.2, 22.2) der verschiedenen Generatormodule für eine Neutralelektrode jeweils elektrisch miteinander verbunden werden können, indem zwei der drei Schalter (28, 30, 32) geschlossen werden und das für den Serienmodus ein Ausgangsanschluss (20.1, 22.1, 20.2, 22.2) für eine Arbeitselektrode eines Generatormoduls und ein Ausgangsanschluss (20.1, 22.1, 20.2, 22.2) für eine Neutralelektrode eines anderen Generatormoduls elektrisch miteinander verbunden werden können, indem der dritte Schalter (28, 30, 32) geschlossen wird, wobei für den Multi-Instrument-Modus alle drei Schalter (28, 30, 32) geöffnet sind.

4. Elektrochirurgie-Generator (10) nach Anspruch 3, **dadurch gekennzeichnet, dass** die Steuereinheit (26) zu einem dem gewählten Betriebsmodus entsprechenden Umschalten der wenigstens drei Schalter (28, 30, 32) mit diesen wirkverbunden ist.

5. Elektrochirurgie-Generator (10) nach wenigstens einem der Ansprüche 1 bis 4, **dadurch gekennzeichnet, dass** die Steuereinheit (26) zum Steuern der HF-Generatoreinheiten (12.1, 12.2) mit jeder der wenigstens zwei HF-Generatoreinheiten (12.1, 12.2) wirkverbunden ist.

6. Elektrochirurgie-Generator (10) nach wenigstens einem der Ansprüche 1 bis 5, **dadurch gekennzeichnet, dass** die Steuereinheit (26) ausgebildet ist, zwei Varianten des Multi-Instrument-Modus bereitzustellen, nämlich eine erste Variante, in der die Ausgangsanschlüsse (22.1; 22.2) zum Anschließen einer Neutralelektrode nicht elektrisch miteinander verbunden sind und eine zweite Variante, in der die Ausgangsanschlüsse (22.1; 22.2) zum Anschließen einer Neutralelektrode elektrisch miteinander verbunden sind.

7. Elektrochirurgie-Generator (10) nach wenigstens einem der Ansprüche 1 bis 6, **dadurch gekennzeichnet, dass** die wenigstens zwei Ausgangsanschlüsse (22.1; 22.2) zum Anschließen einer Neutralelektrode miteinander verbunden sind.

8. Elektrochirurgie-Generator (10) nach wenigstens einem der Ansprüche 1 bis 7, **dadurch gekennzeichnet, dass** die Ausgangstransformatoren (14; 14.2) jeweils primärseitig mit einer der HF-Generatoreinheiten (12.1; 12.2) derart verbunden sind, das die jeweilige HF-Generatoreinheit (12.1; 12.2) zusammen mit dem zugehörigen Ausgangstransformator (14.1; 14.2) einen jeweiligen Schwingkreis bildet, wobei eine Primärwicklung (16.1; 16.2) des jeweiligen Ausgangstransformators (14.1; 14.2) Teil des entsprechenden Schwingkreises ist.

9. Elektrochirurgie-Generator (10) nach wenigstens einem der Ansprüche 2 bis 8, **dadurch gekennzeichnet, dass** in dem Multi-Elektroden-Modus die wenigstens zwei Ausgangsanschlüsse zum Anschließen einer Neutralelektrode elektrisch miteinander verbunden sind und an die Ausgangsanschlüsse zum Anschließen einer Neutralelektrode eine Elektrode eines Elektrochirurgie-Instruments angeschlossen ist.

10. Elektrochirurgie-Generator (10) nach wenigstens einem der Ansprüche 1 bis 9, **dadurch gekennzeichnet, dass** die Steuereinheit ausgebildet ist, die Phasenlage zwischen den von den wenigstens zwei HF-Generatormodulen (24.1, 24.2) abgegebenen Strömen bzw. Spannungen zu steuern.

11. Elektrochirurgie-Generator (10) nach wenigstens einem der Ansprüche 1 bis 10, **dadurch gekennzeichnet, dass** die HF-Generatormodule (24.1, 24.2) so ausgelegt sind, dass die maximale Ausgangsspannung je Generator-Modul 2500 Vₚₑₐₖ beträgt.

12. Elektrochirurgie-Generator (10) nach wenigstens einem der Ansprüche 1 bis 11, **dadurch gekennzeichnet, dass** die HF-Generatormodule (24.1, 24.2) so ausgelegt sind, dass der maximale Ausgangsstrom 4,5 Aᵣₘₛ beträgt.

13. Elektrochirurgie-Generator (10) nach wenigstens einem der Ansprüche 1 bis 12, **dadurch gekennzeichnet, dass** die HF-Generatormodule (24.1, 24.2) so ausgelegt sind, dass deren Frequenzbereich 40 kHz bis 1 MHz umfasst.

## Claims

1. An electrosurgical generator (10), comprising at least two HF generator units (12.1; 12.2) and at least two output transformers (14; 14.2), each having a primary winding (16.1; 16.2) and a secondary winding (18.1; 18.2), of which one of the primary windings (16.1; 16.2) is in each case connected to one of the HF generator units (12.1 ; 12.2) and one of the secondary windings (18.1; 18.2) (14.1; 14.2) is in each case connected to two output terminals (20.1, 22.1; 20.2, 22.2) for connecting an electrosurgical instrument, wherein one of the two output terminals (20.1; 20.2) is in each case provided for connecting a working electrode and the respective other one of the two output terminals (22.1; 22.2) is provided for connecting a neutral electrode, wherein, together with the associated output transformer (14.1; 14.2) and the corresponding output terminals (20.1 , 22.1; 20.2, 22.2), one HF generator unit (12.1; 12.2) respectively forms an HF generator module (24.1; 24.2), wherein the HF generator modules (24.1; 24.2) are connected to a control unit (26), that is configured to at least provide the following operating modes:
- a parallel mode in which the at least two output transformers (14.1, 14.2) are connected in parallel so that, via the output terminals (20.1, 22.1; 20.2, 22.2), an electrosurgical instrument can be operated with a higher output current than the current that would be able to be provided by one HF generator module (24.1; 24.2) alone,
- a series mode in which the at least two output transformers (14.1, 14.2) are connected in series so that, via the output terminals (20.1, 22.1), an electrosurgical instrument can be operated with a higher output voltage than the voltage that would be able to be provided by one HF generator module (24.1; 24.2) alone, and
- a multi instrument mode in which the output transformers (14.1, 14.2) are each connected independently of each other to output terminals for respectively one electrosurgical instrument.

2. An electrosurgical generator (10) according to claim 1, **characterized in that** the electrosurgical generator (10) with its control unit (26) is adapted to additionally provide a multi-electrode mode, in which an output terminal of a generator module is electrically connected to an output terminal another generator module, which results in a total of three output terminals to each of which an electrode of an electrosurgical instrument is connected.

3. An electrosurgical generator (10) according to claim 1 or 2, **characterized by** at least three switches (28, 30,32), that are connected to the output terminals (20.1, 22.1, 20.2, 22.2) in such a way that, for the parallel mode, through the closing of two of the three switches (28, 30,32), two output terminals (20.1, 22.1, 20.2, 22.2) of different generator modules for a working electrode and two output terminals (20.1, 22.1, 20.2, 22.2) of different generator modules for a neutral electrode can respectively be electrically connected to each other, and further **characterized in that**, for the series mode, through the closing of the third switch (28, 30, 32), an output terminal (20.1, 22.1, 20.2, 22.2) for a working electrode of a generator module and an output terminal (20.1, 22.1, 20.2, 22.2) for a neutral electrode of another generator module can be electrically connected to each other, wherein, for the multi-instrument mode, all three switches (28, 30, 32) are open.

4. An electrosurgical generator (10) according to claim 3, **characterized in that** the control unit (26) is operatively connected to at least three switches (28, 30, 32) in order to switch them in accordance with the selected operating mode.

5. An electrosurgical generator (10) according to at least one of claims 1 to 4, **characterized in that** the control unit (26) for controlling the HF generator units (12.1, 12.2) is operatively connected to each of the at least two HF generator units (12.1, 12.2).

6. An electrosurgical generator (10) according to at least one of claims 1 to 5, **characterized in that** the control unit (26) is configured to provide two versions of the multi-instrument mode, namely a first version in which the output terminals (22.1; 22.2) for connecting a neutral electrode are not electrically connected to each other, and a second version in which the output terminals (22.1; 22.2) for connecting a neutral electrode are electrically connected to each other.

7. An electrosurgical generator (10) according to at least one of claims 1 to 6, **characterized in that** the at least two output terminals (22.1; 22.2) for connecting a neutral electrode are connected to each other.

8. An electrosurgical generator (10) according to at least one of claims 1 to 7, **characterized in that** the output transformers (14; 14.2) are in each case on their primary side connected to one of the HF generator units (12.1; 12.2) in such a way that, together with the associated output transformer (14.1; 14.2), the respective HF generator unit (12.1 ; 12.2) forms a respective resonant circuit, wherein a primary winding (16.1; 16.2) of the respective output transformer (14.1; 14.2) is part of the respective resonant circuit.

9. An electrosurgical generator (10) according to at least one of claims 2 to 8, **characterized in that**, in the multi-electrode mode, the at least two output terminals for connecting a neutral electrode are electrically connected, and **in that** an electrode of an electrosurgical instrument is connected to the output terminals for connecting a neutral electrode.

10. An electrosurgical generator (10) according to at least one of claims 1 to 9, **characterized in that** the control unit is configured to control the phase position between the currents and/or voltages output by the at least two HF generator modules (24.1, 24.2).

11. An electrosurgical generator (10) according to at least one of claims 1 to 10, **characterized in that** the HF generator modules (24.1, 24.2) are configured such that the maximum output voltage per generator module is 2,500 Vₚₑₐₖ.

12. An electrosurgical generator (10) according to at least one of claims 1 to 11, **characterized in that** the HF generator modules (24.1, 24.2) are configured such that the maximum output current is 4.5 Aᵣₘₛ.

13. An electrosurgical generator (10) according to at least one of claims 1 to 12, **characterized in that** the HF generator modules (24.1, 24.2) are configured such that their frequency range comprises 40 kHz to 1 MHz.

## Revendications

1. Générateur d'électrochirurgie (10) comprenant au moins deux unités de générateur HF (12.1 ; 12.2) et au moins deux transformateurs de sortie (14 ; 14.2) avec chacun un enroulement primaire (16.1 ; 16.2) et un enroulement secondaire (18.1 ; 18.2), dont l'un des enroulements primaires (16.1 ; 16.2) est relié à l'une des unités de générateur HF (12.1 ; 12.2) et l'un des enroulements secondaires (18.1 ; 18.2) (14.1 ; 14.2) est relié à deux bornes de sortie (20.1, 22.1 ; 20.2, 22.2) pour le raccordement d'un instrument d'électrochirurgie, l'une des deux bornes de sortie (20.1 ; 20.2) étant prévue pour le raccordement d'une électrode de travail et l'autre (22.1 ; 22.2) pour le raccordement d'une électrode neutre, chacune des unités de générateur HF (12.1 ; 12.2) formant avec le transformateur de sortie associé (14.1 ; 14.2) et les bornes de sortie correspondantes (20.1, 22.1 ; 20.2, 22.2) un module de générateur HF (24.1 ; 24.2), les modules de générateur HF (24.1 ; 24.2) étant reliés à une unité de commande (26) étant conçus pour fournir au moins les modes de fonctionnement suivants :
- un mode parallèle, dans lequel au moins les deux transformateurs de sortie (14.1, 14.2) sont connectés en parallèle, de sorte qu'un instrument d'électro-chirurgie puisse être exploité via les bornes de sortie (20.1, 22.1 ; 20.2, 22.2) avec un courant de sortie supérieur à celui qui peut être fourni par un seul module de générateur HF (24.1 ; 24.2),
- un mode série, dans lequel au moins les deux transformateurs de sortie (14.1, 14.2) sont connectés en série, de sorte qu'un instrument d'électrochirurgie puisse être exploité via les bornes de sortie (20.1 ; 22.2) avec une tension de sortie supérieure à celle qui peut être fournie par un seul module de générateur HF (24.1 ; 24.2), et
- un mode multi-instruments, dans lequel les transformateurs de sortie (14.1 ; 14.2) sont reliés indépendamment les uns des autres à des bornes destinées chacune à un instrument d'électrochirurgie.

2. Un générateur d'électrochirurgie (10) selon la revendication 1, se **caractérise en ce que** le générateur d'électrochirurgie est conçu (10) avec son unité de commande (26) pour fournir un mode multi-électrodes supplémentaire, dans lequel une borne de sortie d'un module de générateur est reliée électriquement à une borne de sortie d'un autre module de générateur, de manière à ce qu'il en résulte au total trois bornes de sortie, auxquelles est raccordée respectivement une électrode d'un instrument d'électrochirurgie.

3. Un générateur d'électrochirurgie (10) selon la revendication 1 ou 2, se **caractérise par** au moins trois commutateurs (28, 30, 32) qui sont reliés aux bornes de sortie (20.1, 22.1, 20.2, 22.2) de manière à ce que, pour le mode parallèle, deux bornes de sortie (20.1, 22.1, 20.2, 22.2) de différents modules de générateur pour une électrode de travail et deux bornes de sortie (20.1, 22.1, 20.2, 22.2) de différents modules de générateur pour une électrode neutre puissent être respectivement reliés électriquement entre elles en fermant deux des trois commutateurs (28, 30, 32) et que, pour le mode série, une borne de sortie (20.1, 22.1, 20.2, 22.2) pour une électrode de travail d'un module de générateur et une borne de sortie (20.1, 22.1, 20.2, 22.2) pour une électrode neutre d'un autre module de générateur puissent être reliés électriquement l'une à l'autre en fermant le troisième commutateur (28, 30, 32), les trois commutateurs (28, 30, 32) étant ouverts pour le mode multi-instruments.

4. Un générateur d'électrochirurgie (10) selon la revendication 3, se **caractérise en ce que** l'unité de commande (26) est reliée fonctionnellement aux trois commutateurs (28, 30, 32) pour une commutation des au moins trois commutateurs correspondant au mode de fonctionnement sélectionné.

5. Un générateur d'électrochirurgie (10) selon au moins l'une des revendications 1 à 4, se **caractérise en ce que** l'unité de commande (26) destinée à commander les unités de générateur HF (12.1, 12.2) est reliée fonctionnellement à chacune des au moins deux unités de générateur HF (12.1, 12.2).

6. Un générateur d'électrochirurgie (10) selon au moins l'une des revendications 1 à 5, se **caractérise en ce que** l'unité de commande (26) est conçue pour fournir deux variantes du mode multi-instruments, à savoir une première variante dans laquelle les bornes de sortie (22.1 ; 22.2) pour le raccordement d'une électrode neutre ne sont pas reliées électriquement entre elles et une deuxième variante dans laquelle les bornes de sortie (22.1 ; 22.2) pour le raccordement d'une électrode neutre sont électriquement reliées entre elles.

7. Un générateur d'électrochirurgie (10) selon au moins l'une des revendications 1 à 6, se **caractérise en ce qu'**au moins les deux bornes de sortie (22.1 ; 22.2) sont reliées entre elles pour le raccordement d'une électrode neutre.

8. Un générateur d'électrochirurgie (10) selon au moins l'une des revendications 1 à 7, se **caractérise en ce que** les transformateurs de sortie (14 ; 14.2) sont reliés respectivement côté primaire à l'une des unités de générateur HF (12.1 ; 12.2) de sorte que l'unité de générateur HF respective (12.1 ; 12.2) forme un circuit oscillant avec le transformateur de sortie associé (14 ; 14.2), un enroulement primaire (16.1 ; 16.2) du transformateur de sortie respectif (14.1 ; 14.2) faisant partie du circuit oscillant correspondant.

9. Un générateur d'électrochirurgie (10) selon au moins l'une des revendications 2 à 8, se **caractérise en ce que**, dans le mode multi-électrodes, les au moins deux bornes de sortie pour le raccordement d'une électrode neutre sont reliées électriquement entre elles et une électrode d'un instrument d'électrochirurgie est raccordée aux bornes de sortie pour le raccordement d'une électrode neutre.

10. Un générateur d'électrochirurgie (10) selon au moins l'une des revendications 1 à 9, se **caractérise en ce que** l'unité de commande est conçue pour commander la relation de phase entre les courants et/ou tensions générés par les au moins deux modules de générateur HF (24.1, 24.2).

11. Un générateur d'électrochirurgie (10) selon au moins l'une des revendications 1 à 10, se **caractérise en ce que** les modules de générateur HF (24.1, 24.2) sont conçus de sorte que la tension de sortie maximale par module de générateur s'élève à 2 500 Vcrête.

12. Un générateur d'électrochirurgie (10) selon au moins l'une des revendications 1 à 11, se **caractérise en ce que** les modules de générateur HF (24.1, 24.2) sont conçus de sorte que le courant maximal de sortie s'élève à 4,5 Arms.

13. Un générateur d'électrochirurgie (10) selon au moins l'une des revendications 1 à 12, se **caractérise en ce que** les modules de générateur HF (24.1, 24.2) sont conçus de sorte que leur plage de fréquences soit comprise entre 40 kHz et 1 MHz.
